(19) Europäisches Patentamt
European Patent Office
Office européen des brevets

(11) Veröffentlichungsnummer: **0 001 417**
**A1**

# (12) EUROPÄISCHE PATENTANMELDUNG

(21) Anmeldenummer: **78100944.4**

(22) Anmeldetag: **20.09.78**

(51) Int. Cl.²: **C 07 C 17/02**, C 07 C 19/02

(30) Priorität: **30.09.77 DE 2743975**

(43) Veröffentlichungstag der Anmeldung: **18.04.79 Patentblatt 79/8**

(84) Benannte Vertragsstaaten: **BE CH DE FR GB NL**

(71) Anmelder: **Bayer Aktiengesellschaft, Zentralbereich Patente,Marken und Lizenzen Bayerwerk, D-5090 Leverkusen 1 (DE)**

(72) Erfinder: **Hartung, Sigurd, Dr., Neue Kempener Strasse 246, D-5000 Köln 60 (DE)**

## (54) Verfahren zur Herstellung von 1,2-Dichloräthan.

(57) Die Erfindung betrifft ein Verfahren zur Herstellung eines praktisch vinylchloridfreien 1,2-Dichloräthans durch Umsetzung von Athylen und Chlor in einem Lösungsmittel bei an sich sonst üblicher Katalysatorzugabe, bei dem man Chlor und Äthylen in einem Lösungsmittel bei erhöhter Temperatur ohne Zusatz eines Katalysators umsetzt.

EP 0 001 417 A1

0001417



BAYER AKTIENGESELLSCHAFT
Zentralbereich
Patente, Marken und Lizenzen

5090 Leverkusen, Bayerwerk
Mn/by

1,2-Dichloräthan

BEZEICHNUNG GEÄNDERT
siehe Titelseite

Die Erfindung betrifft ein Verfahren zur Herstellung von praktisch Vinylchlorid-freiem 1,2-Dichloräthan ohne Zusatz eines Halogenübertragungskatalysators.

Es ist bekannt, daß man 1,2-Dichloräthan erhält, wenn man Chlor und Äthylen in Gegenwart von Halogenübertragungskatalysatoren, wie Eisen-III-chlorid, Wismut-III-chlorid, Tellur-(IV)-chlorid und/oder Zinn-IV-chlorid, umsetzt (DT-OS 2 540 257).

Ebenfalls ist bekannt, daß ein zu niedriger Katalysatorgehalt unerwünschte Substitutionsreaktionen fördert und die Umsetzung verlangsamt, während eine Überdosierung des Katalysators Kosten für den überflüssigen Mehrverbrauch und die anschließende Auswaschung verursacht und wegen der geringen Löslichkeit der Metallsalze im Lösungsmittel auch zu Verstopfungen führen kann (DT-OS 2 540 291 (Seite 4)).

Es wurde ein Verfahren zur Herstellung eines praktisch Vinylchlorid-freien 1,2-Dichloräthans durch Umsetzung von Äthylen und Chlor in einem Lösungsmittel bei an sich sonst üblicher Katalysatorzugabe gefunden, das dadurch gekennzeichnet ist,

daß man Äthylen und Chlor in einem Lösungsmittel bei erhöhter Temperatur ohne Zusatz eines Katalysators umsetzt.

Das erfindungsgemäße Verfahren kann durch die folgende Reaktionsgleichung erläutert werden.

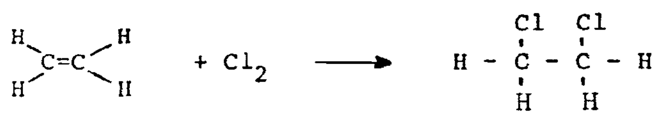

Unter einem praktisch Vinylchlorid-freien 1,2-Dichloräthan nach dem erfindungsgemäßen Verfahren versteht man ein 1,2-Dichloräthan, das im allgemeinen weniger als 1 ppm Vinylchlorid enthält.

Es kann sowohl reines als auch technisches Chlor in das erfindungsgemäße Verfahren eingesetzt werden. Technisches Chlor kann als Verunreinigungen beispielsweise Stickstoff, Kohlendioxid, Wasserstoff, Wasser, Chlorwasserstoff, Brom, Jod und Sauerstoff enthalten. Im allgemeinen setzt man in das erfindungsgemäße Verfahren ein Chlor mit einem Chlorgehalt von 85 bis 100 Vol.-%, bevorzugt 90 bis 95 Vol.-%, ein.

Ebenso kann sowohl reines als auch technisches Äthylen in das erfindungsgemäße Verfahren eingesetzt werden. Technisches Äthylen kann als Verunreinigungen beispielsweise niedere Kohlenwasserstoffe, wie Methan, Äthan und Propan, Acetylen, Stickstoff, Kohlendioxid und Wasser enthalten. Im allgemeinen setzt man in das erfindungsgemäße Verfahren ein Äthylen mit einem Äthylengehalt von 95 bis 100 Vol.-%, bevorzugt 96 bis 99,5 Vol.-%, ein.

Nach dem erfindungsgemäßen Verfahren werden Chlor und Äthylen in etwa äquivalenten Mengenverhältnissen eingesetzt. Im allgemeinen setzt man 1 Molteil Chlor mit 0,95 bis 1,05 Molteilen Äthylen um.

Das erfindungsgemäße Verfahren wird bei erhöhter Temperatur, im allgemeinen im Temperaturbereich von 20 bis 80°C durchgeführt. Bevorzugt führt das erfindungsgemäße Verfahren bis zum Temperaturbereich von 40 bis 60°C.

Das erfindungsgemäße Verfahren wird im allgemeinen in einer offenen Reaktionsapparatur, also praktisch bei Normaldruck, durchgeführt. Selbstverständlich sind kleine Druckschwankungen etwa von 1 bis 5 bar möglich.

Als Lösungsmittel für das erfindungsgemäße Verfahren seien 1,2-Dichloräthan, aliphatische Kohlenwasserstoffe, wie n-Hexan, Isooctan, Isododecan und Paraffingemische, aromatische Kohlenwasserstoffe, wie Benzol, Toluol, Xylole, Äthylbenzol, Cumol und Halogenkohlenwasserstoffe, wie Bromäthan, Dibromäthan, Trichloräthan, Dichlorbutan, Chlorpropan, Chlorbutan genannt. Bevorzugtes Lösungsmittel ist 1,2-Dichloräthan. Insbesondere bevorzugt bei kontinuierlicher Arbeitsweise in einem Kreislaufreaktor wird ein Teil des entstandenen 1,2-Dichloräthans zurückgeführt und als Lösungsmittel der Ausgangskomponenten eingesetzt.

In einer besonderen Ausführungsform des erfindungsgemäßen Verfahrens beträgt die Verweilzeit (Volumen des Reaktionsraumes [$m^3$] pro Volumen des entstandenen 1,2-Dichloräthans [$m^3$ / Std.]) des Chlors und des Äthylens in der Reaktionsapparatur 2 bis 7 Stunden, bevorzugt 2,5 bis 5 Stunden, und der Verwirbelungsfaktor (Pumpenleistung $[m^3$ / Std.] pro Volumen des entstandenen 1,2-Dichloräthans $[m^3$ / Std.]) von 20 bis 500, bevorzugt von 300 bis 400.

Als Materialien für die Reaktionsapparatur seien genannt: Glas, Keramik, Polytetrafluoräthylen, Graphit, Kunstharze, beispielsweise aus Phenol und Formaldehyd, Nickel und Nickellegierungen, Eisen, Zirkon, Tantal, Titan und austenitische Stähle.

In einer bevorzugten Ausführungsform des erfindungsgemäßen Verfahrens verwendet man eine Reaktionsapparatur die Bestandteile aus Eisen enthält. Bei Verwendung einer Eisenbestandteile enthaltenden Reaktionsapparatur enthält das Reaktionsgemisch praktisch kein, höchstens 25 ppm, bevorzugt weniger als 2 ppm, Eisenionen.

Das erfindungsgemäße Verfahren kann diskontinuierlich und kontinuierlich durchgeführt werden.

Eine Ausführungsform des erfindungsgemäßen Verfahrens sei beispielhaft mit Hilfe von Figur 1 erläutert:

In dem Reaktionskreislauf (3) wird das Lösungsmittel, z.B. 1,2-Dichloräthan, mit Hilfe der Pumpe (5) umgewälzt. Durch die Eingänge (1) und (2) werden Chlor und Äthylen eingeleitet. Die Reaktionswärme wird im Wärmetauscher (4) abgeleitet. Am Ausgang (6) ist die Reaktionsapparatur offen und gegebenenfalls über ein Kühlsystem mit einer Abgasleitung verbunden.

Das Reaktionsprodukt wird bei (7), gegebenenfalls über einen Nachreaktor, in die Destillationskolonne (8) geleitet. Am Kopf der Kolonne (9) wird reines 1,2-Dichloräthan entnommen. Im Sumpf der Kolonne werden Nebenprodukte, beispielsweise höherchloriertes Äthylen, abgetrennt.

Es ist überraschend, daß das erfindungsgemäße Verfahren ohne Zugabe eines sonst üblichen Katalysators durchgeführt werden kann.

Vorteilhafterweise entsteht nach dem erfindungsgemäßen Verfahren praktisch kein Vinylchlorid, das sonst in aufwendigen Reinigungsoperationen abgetrennt werden müßte. Vinylchlorid in der Abluft ist bekanntlich eine Belastung der Umwelt.

Üblicherweise wird ein Katalysator bei der Herstellung von 1,2-Dichloräthylen in aufwendiger Weise über Teilströme und Filtereinsätze geführt (z.B. DT-OS 2 540 291). Vorteilhafterweise entfallen nach dem erfindungsgemäßen Verfahren solche Arbeitsweisen.

Da kein Katalysator nach dem erfindungsgemäßen Verfahren zugegeben wird, erspart man sich vorteilhafterweise die aufwendige Abtrennung des Katalysators aus dem Endprodukt.

1,2-Dichloräthan ist ein Zwischenprodukt für Antiklopfmittel, Äthylendiamin und Äthylenpolyamine.

<u>Beispiele:</u>

Die einzelnen Beispiele werden in der im folgenden beschriebenen und mit Hilfe von Figur 1 erläuterten Reaktionsapparatur durchgeführt:

In dem Reaktionskreislauf (3) wird 1,2-Dichloräthan mit Hilfe der Pumpe (5) umgewälzt. Durch die Eingänge (1) und (2) werden Chlor und Äthylen eingeleitet. Die Reaktionswärme wird im Wärmetauscher (4) abgeleitet. Am Ausgang (6) ist die Reaktionsapparatur offen und mit einer auf minus 16 °C gekühlten Kühlfalle verbunden, in der gegebenenfalls entstandenes Vinylchlorid kondensiert wird.

Das Reaktionsprodukt wird bei (7), gegebenenfalls über einen Nachreaktor, in die Destillationskolonne (8) geleitet. Am Kopf der Kolonne (9) wird reines 1,2-Dichloräthan entnommen. Im Sumpf der Kolonne werden Nebenprodukte, beispielsweise höherchloriertes Äthylen, abgetrennt.

<u>Beispiel 1</u>

| | |
|---|---|
| Werkstoff: | Glas und Kunststoffteile (produktführende Teile enthalten kein Eisen) |
| Chlorgas: | 34,5 l / Std. (Chlorgehalt 95 %) |
| Äthylen: | 32,6 l / Std. (Äthylengehalt 99,5 %) |
| Lösungsmittel: | 1,2-Dichloräthan |
| Reaktionstemperatur: | 45 bis 50 °C |
| Pumpenleistung: | 40 [l / Std.] |

Verweilzeit: 3,8 Stunden
Verwirbelungsfaktor: 307,6
Ausbeute: 130 g 1,2-Dichloräthan pro Stunde, das entspricht einer Ausbeute von 99,1 %, bezogen auf Äthylen
Vinylchloridgehalt des 1,2-Dichloräthans: 0,2 ppm
Vinylchloridgehalt im Abgas: 2,5 ppm

Beispiel 2

| | |
|---|---|
| Werkstoff: | Reaktionsapparatur besteht im wesentlichen aus Eisenteile enthaltenden Elementen |
| Chlorgas: | 421 $m^3$/Std |
| Äthylen: | 392 $m^3$/Std |
| Lösungsmittel: | 1,2-Dichloräthan |
| Reaktionstemperatur: | 55°C |
| Pumpenleistung: | 65 $m^3$/Std |
| Verweilzeit: | 3 Stunden |
| Verwirbelungsfaktor: | 40,6 |
| Ausbeute: | 1600 kg Dichloräthan, das entspricht einer Ausbeute, bezogen auf Äthylen von 99,88 %. |
| Eisengehalt im Lösungsmittel: | 20 ppm |
| Vinylchloridgehalt des 1,2-Dichloräthans | 0,6 ppm |
| Vinylchloridgehalt im Abgas: | 1,6 ppm |

<u>Beispiel 3</u> (Vergleichsbeispiel zu Beispiel 2)

| | |
|---|---|
| Werkstoff: | Reaktionsapparatur besteht im wesentlichen aus Eisenteilen enthaltenden Elementen |
| Chlorgas: | 184 $m^3$/Std |
| Äthylen: | 178,5 $m^3$/Std |
| Lösungsmittel: | Dichloräthan |
| Katalysatorgehalt im Lösungsmittel durch Zugabe von Eisen (III)-chlorid: | 200 ppm |
| Reaktionstemperatur: | 60°C |
| Pumpenleistung: | 65 $m^3$/St |
| Verweilzeit: | 7 Stunden |
| Verwirbelungsfaktor: | 91,5 |
| Ausbeute: | 700 kg 1,2-Dichloräthan, das entspricht einem Umsatz von 97,9 % bezogen auf Äthylen |
| Eisengehalt im Endprodukt: | 200 ppm |
| Vinylchloridgehalt des 1,2-Dichloräthans: | 40 ppm |
| Vinylchloridgehalt im Abgas: | 15 ppm |

Patentansprüche

1. Verfahren zur Herstellung eines praktisch Vinylchlorid-freien 1,2-Dichloräthans durch Umsetzung von Äthylen und Chlor in einem Lösungsmittel bei an sich sonst üblicher Katalysatorzugabe, dadurch gekennzeichnet, daß man Chlor und Äthylen in einem Lösungsmittel bei erhöhter Temperatur ohne Zusatz eines Katalysators umsetzt.

2. Verfahren nach Anspruch 1, dadurch gekennzeichnet, daß es bei einer Verweilzeit des Chlors und des Äthylens in der Reaktionsapparatur von 2 bis 7 Stunden und mit einem Verwirbelungsfaktor von 20 bis 500 durchgeführt wird.

3. Verfahren nach Anspruch 1 und 2, dadurch gekennzeichnet, daß die Umsetzung in einer Eisenteile enthaltenden Reaktionsapparatur durchgeführt wird.

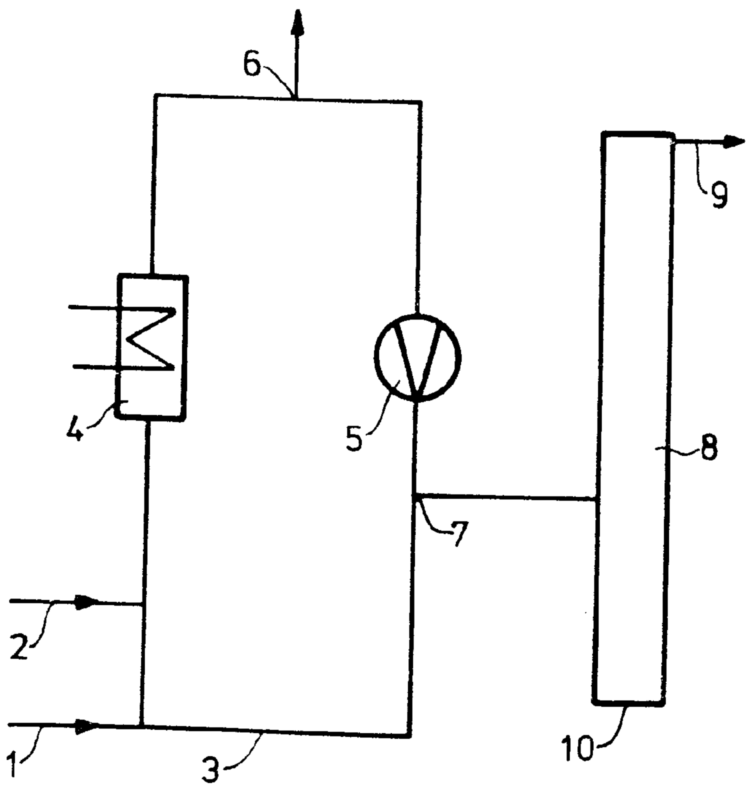


FIG. 1

0001417

Europäisches Patentamt

# EUROPÄISCHER RECHERCHENBERICHT

Nummer der Anmeldung
EP 78 10 094

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| D | DE - A - 2 540 291 (HOECHST AG)<br>* Spalte 3, Anfang bis Seite 4, Mitte *<br>--<br>FR - A - 501 495 (UNION CARBIDE CO)<br>* Seite 2, Zeilen 26 bis 50 *<br>--<br>ULLMANNS ENCYCLOPAEDIE DER TECHNISCHEN CHEMIE, 4. Auflage, Band 9, (1975), Verlag Chemie, Weinheim/Bergstr., Seiten 426-8<br>* Seite 427 *<br>--<br>CHEMICAL ENGENEERING, April 29, 1974, Seiten 143-146<br>* Seite 146, Spalte 2 "Ethylene Dichloride" *<br>---- | 1<br><br><br>1<br><br><br>1<br><br><br>1 | C 07 C 17/02<br>19/02<br><br>RECHERCHIERTE SACHGEBIETE (Int. Cl.²)<br>C 07 C 17/02<br>19/02<br><br>KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument<br>&: Mitglied der gleichen Patentfamilie, übereinstimmendes Dokument |
| X | Der vorliegende Recherchenbericht wurde für alle Patentansprüche erstellt. | | |

| Recherchenort | Abschlußdatum der Recherche | Prüfer |
|---|---|---|
| Den Haag | 04-12-1978 | BESLIER |

EPA form 1503.1 06.78